# EUROPEAN PATENT APPLICATION

(11) **EP 2 209 002 A1**
(43) Date of publication of application: **21.07.2010**
(21) Application number: 09150634.5
(22) Date of filing: 15.01.2009
(51) Int. Cl.: G01N 33/68

(54) **Assembly of absolutely quantified peptide and phosphopeptide solutions via element mass spectrometry**

(71) Applicant: DKFZ Deutsches Krebsforschungszentrum, 69120 Heidelberg (DE)
(72) Inventor: Lehmann, Wolf-Dieter, 69120 Heidelberg (DE); Zinn, Nico, 64625 Bensheim (DE); Hahn, Bettina, 68535 Edingen (DE)
(74) Representative: Huhn, Michael

(57) **Abstract**

The present invention relates generally to the fields of analytics of proteins and peptides. More particularly, the invention concerns generation of an absolutely quantified reference peptide or protein solution via element mass spectrometry. Furthermore, the invention relates to the absolute quantification of phosphopeptides and peptides by the use of an absolutely quantified peptide solution.

### SEQUENCE LISTING

<110> DKFZ Deutsches Krebsforschungszentrum, Stiftung des öffentlichen Rechts
<120> Absolute quantification of phosphopeptides via element mass spectrometry for the production of absolutely quantified peptide solutions via dephosphorylation
<130> DK66160EP
<160> 3
<170> PatentIn version 3.5
<210> 1
   <211> 13
   <212> PRT
   <213> artificial
<220>
   <223> for proof of principle
<400> 1
   His Thr Asp Asp Glu Met Thr Gly Tyr Val Ala Thr Arg
   1 5
<210> 2
   <211> 9
   <212> PRT
   <213> artificial
<220>
   <223> for proof of principle
<400> 2
   Tyr Arg Ser Val Thr Pro Cys Asp Met
   1 5
<210> 3
   <211> 9
   <212> PRT
   <213> artificial
<220>
   <223> for proof of principle
<400> 3
   Tyr Arg Ser Val Thr Pro Cys Asp Met
   1 5

## Description

The present invention relates generally to the fields of analytics of proteins and peptides. More particularly, the invention concerns generation of absolutely quantified peptide or protein solutions via element mass spectrometry used as internal standards in downstream proteomics experiments. Furthermore, the invention relates to the absolute quantification of phosphopeptides.

With the completion of the Human Genome Project, the emphasis is shifting to examining the protein complement in the human organism. This has given a rise to the science of proteomics, the study of the whole amount of the proteins produced by a cell type of an organism. At the same time, there has been a revival of interest in proteomics in many prokaryotes and eukaryotes as well.

The term proteome refers to all proteins expressed by a genome and comprises the identification of proteins in the body and the detection of their physiological and pathophysiological function. The about 30.000 genes, defined by the Human Genome Project translate into 30.000 up to 1 million of proteins, when alternate splicing and posttranslational modifications are considered. While a specific genome remains unchanged to a high extent, the proteins in any particular cell change dramatically as genes are turned on and off, e.g. in response to extracellular stimulation.

As a reflection of the dynamic nature of the proteome, the term "functional proteome" is preferred by some scientists, which describes the entity of proteins produced by a cell at a certain time point. Finally, it is believed, that through proteomics, new disease markers and drug targets can be identified that will help to design new products to prevent, diagnose and treat disease.

Primary themes in proteomics are protein identification and the differentiation of physiological or pathological protein expression levels (comparative proteomics). The long-term goal of being able to define the entire proteome of a cell is still unrealized, but the characterization of many thousands of proteins in a single analysis is now attainable.

For proteomics to become a platform technology serving the emergent field of systems biology, there is a need for enhancement of quantification. Most comparative proteomic studies deliver a relative quantification, expressing the changes in concentrations of a protein in the context of a different cellular stage. However, the goal is to define the cellular concentrations of proteins absolutely, whether as molarities or as numbers of molecules per cell. Absolute quantification, which poses one of the greatest challenges in proteomics, draws on well-established concepts in analytical chemistry and requires either external standards or internal standards. Quantification by immunological methods is typically performed by external standardization e.g. on position-addressable antibody arrays. The second approach, reliant on internal standardization, is based on mass spectrometry (MS), wherein highly selective detection of molecular or fragment ions is performed.

In the most MS-analysis, stable isotope labeled variants of the analytes are used as internal standards. The key principle is that mass spectrometric signal intensities can be converted into absolute quantities of analytes by referencing to an authentic standard present in known concentration. The direct application of this approach to intact proteins is impractical and it is common to adopt the surrogate principle to quantify by referencing to a unique proteolytic peptide derived from the protein of interest.

Synthetic stable isotope labeled peptides used as internal standards have been named "AQUA" peptides (Absolute Quantification) (Gerber PNAS 100 (2003), 1940-1945). AQUA peptides are quantified by total hydrolysis, derivatisation of the amino acids and their quantification by UV spectroscopy, fluorescence spectroscopy or mass spectrometry. The accuracy of this analysis is dependent on the purity of the sample. Traces of peptides with deletion sequences, salts, metals or detergents can affect the accuracy of results. Conditions applied during hydrolysis are rigorous and destroy some of the amino acids (Met, Cys, Gln, Asn, Trp) and special conditions have to be applied for modified amino acids.

The international patent application WO 03/102220 provides methods to determine the absolute quantity of proteins present in a biological sample. The principle of WO 03/102220 is based on the generation of an ordered array of differently isotopically tagged pairs of peptides, wherein each pair represents a unique protein, as specific protein isoform or a specifically modified form of a protein. One element of the peptide pairs is a synthetically generated, external standard, and the other element of the pair is a peptide generated by enzymatic digestion of the proteins in a sample mixture. For performing the method of WO 03/102220, the standard peptides are calibrated so that absolute concentrations are known and added for comparison and quantification. A sample of interest is also labeled with the same chemical tag as used for the standard peptides except differing in the isotopic label. The signal pairs, which correspond to differently labeled samples and standard peptides are finally observed and related to a list of expected masses based on the particular standard peptides included. The disadvantages of this patent application are that both the sample and the standard peptides need to be specifically labeled separately, increasing the potential for variability between experiments.

Other methods for proteome analysis include quantitative mass spectrometry based on multidimensional peptide separation and isotope-coded affinity tagging of proteins. This method allows only relative quantitation. Also, chip technology using arrays of reagents with known specifity for target proteins, such as antibody arrays or arrays of aptamers, can be used for proteomic analysis. However, the use of such arrays can be limited by the need for selectivity, and for the preservation of the three-dimensional structure of the immobilized proteins.

Each quantitation approach has advantages and disadvantages. Quantification of protein expression ratios by metabolic labeling, such as a stable isotope labeling in cell culture (SILAC) (Oda Y., Huang K., Cross F.R., Cowburn D., Chait B.T., Proc. Natl. Acad. Sci. USA, 1999, Vol. 96, 6591-6596) provides the opportunity to quantify proteins, but has the limitation that primary tissue cannot be analyzed.

Thus, there is still an existing need to develop easy and convenient methods for determination of the absolute concentration and the degree of phosphorylation of a peptide which contain less sources of error and are less time consuming.

Accordingly, an aim of the present invention is to provide an accurate method for determining the degree of phosphorylation and the absolute concentration of a peptide.

This is achieved by a method for determining the degree of phosphorylation of a first peptide which is present in a sample in phosphorylated and/or unphosphorylated form or any mixtures thereof comprising:
(a) obtaining a sample containing said first peptide,
(b) preparing a reference sample containing a second peptide which contains at least one phosphate group and a known degree of phosphorylation and which is an isotope labelled form of the first peptide, and a third peptide that is a completely dephosphorylated and isotope labeled form of the first peptide in a known concentration comprising:
   (ba) providing a phosphorus standard having a known phosphorus concentration,
   (bb) providing the second peptide and combining the second peptide and the phosphorus standard,
   (bc) detecting a signal of the second peptide and of the phosphorus standard in an LC-ICP-MS system,
   (bd) determining the concentration of the second peptide by comparing the respective phosphorus signals,
   (be) providing an aliquot of the second peptide of known concentration obtained in step (bd) and hydrolyzing the phosphoric acid ester bond(s) to obtain the third peptide with known concentration, and
   (bf) combining a known amount of the second peptide quantified in step (bd) and a known amount of the corresponding completely dephosphorylated third peptide obtained in step (be) to obtain the reference (internal standard) sample,
(c) combining the reference sample and the sample containing the first peptide,
(d) detecting the molecular ion and/or fragment ion signals of each of the peptides of the reference sample and the first peptide with an analyzer selected from ESI, LC-ESI and MALDI, and
(e) determining the degree of phosphorylation of the first peptide by comparison of the respective signals.

It was found, that the method according to the invention is a surprisingly easy and sensitive way for generating a reference sample, containing either the isotope labeled non-phosphorylated or phosphorylated analogue of the first peptide or any mixtures thereof. In this way the determination of the absolute amount of each form of the first peptide respectively the degree of phosphorylation of the first peptide is performed. Advantageously, the method according to the invention incorporates fewer steps and thus contains less sources of error compared to existing methods for generating a reference sample which can be used for determining the absolute amount and degree of phosphorylation of peptides.

According to an embodiment of the invention, the degree of phosphorylation and the absolute concentration of the first peptide are determined by comparison of the respective signals in a further step (f) of the method of the invention. Hence, the present invention allows accurate determination of the absolute amount / concentration and degree of phosphorylation of a phosphopeptide. According to this aspect of the invention, a method for the absolute quantification of a phosphopeptide is disclosed, wherein only 0.01 to 0.1 nmol of the first peptide are required.

The term "first peptide" encompasses the unphosphorylated and/or phosphorylated form of a peptide or any mixtures thereof. As used herein, the term "peptide" refers to a peptide or a polypeptide of two or more amino acids. The polypeptide can additionally be modified by naturally occurring modifications, such as posttranslational modifications, including fatty acylation, sulfation, hydroxylation, acetylation, glycosylation, addition of prosthetic groups or cofactors, formation of disulfide bonds, assembly in molecular complexes and the like.

According to an embodiment of the invention, the expression "first peptide" encompasses a derivatized phosphopeptide, too. For peptides without serine (S), threonine (T) and/or tyrosine (Y) residues, the derivatisation comprises the addition of an extension carrying a phosphorylated serine, threonine and/or tyrosine residue. According to this aspect of the invention, the "first peptide" is obtained by the addition of a short, optionally enzyme-cleavable extension carrying a phosphorylated residue (pS, pT and/or pY). The addition can be performed at the N-terminus or at the C-terminus of the peptides, respectively. According to this aspect of the invention, in step (be) of the inventive method, this extension is cleaved by treatment with a protease and the third peptide with known concentration is obtained.

As used herein the term "sample" is intended to mean any biological fluid, cell tissue, organ or portion thereof that includes one or more different molecules, such as nucleic acid, polypeptides, or small molecules. A sample can be a tissue section obtained by a biopsy or a cell placed in or adapted to tissue culture. A sample can also be a biological fluid, specimen such as blood or plasma, cerebrospinal fluid, urine, saliva, seminal plasma, pancreatic juice, and the like. A sample can additionally be a cell extract from any species, including procaryotic and eucaryotic cells as well as viruses. A tissue or biological fluid specimen can be further fractionated, if desired, to a fraction containing particular cell types.

The method of the invention is to produce a reference sample of known concentration for determining the absolute amount and degree of phosphorylation of a phosphopeptide and/or for determining the absolute concentration of a peptide in a sample. The method is based on the identification of a distinct peptide which is unique for a polypeptide or protein which can therefore be used as surrogate to identify the degree of phosphorylation and/or the quantity of this polypeptide or protein in a sample.

Peptides uniquely identifying a protein can be selected experimentally or computationally. Experimentally such peptides are selected from databases that contain all the peptides, preferably phosphopeptides, from a species that have been previously observed, for example, by mass spectrometry experiments. Computationally such peptides are selected by translating the complete genomic sequence or the sequence of all predicted genes and their splice forms into the corresponding amino acid sequences, by applying the rules for cleavage that are predictable with each chemical or enzymatic protein cleavage reagent to these amino acid sequences, and by computing the sequence, mass and other properties for each of the general peptides. From this database of predicted peptides, a suitable selection of peptides that are unique for each target protein, in the context of the invention called first peptide, is the selected.

In the preferred embodiment of the invention, the first peptide has 5 to 100 amino acids, more preferred 5 to 50 amino acids, and most preferred 6 to 20 amino acids.

Once the first peptide is selected, a reference sample can be prepared by synthesizing a second peptide which contains at least one phosphate group and a known degree of phosphorylation and which is an isotope labelled form of the first peptide. The second peptide having an identical chemical structure like the first peptide is made by synthesis using a number of isotopic atoms for example ¹³C, ¹⁵N, ¹⁷O, ¹⁸O or ³⁴S, so that the first and the second peptide can be distinguished using mass spectrometry. Peptide sequences, once selected, are preferably chemically synthesized by solid-phase step by step synthesis. Methods of peptide synthesis are well-known to those skilled in the art. The completely phosphorylated second peptide carries at least one isotopic atom selected from ¹³C, ¹⁵N, ¹⁷O, ¹⁸O or ³⁴S. According to an embodiment of the invention, the second and third peptide contains 3 to 12, preferred 4 to 10 of these isotopic atoms.

The second peptide is subjected to absolute quantification by use of a LC-ICP mass spectrometer. For this purpose a known amount of a phosphorus standard having a known phosphorus concentration is combined with a solution containing the second peptide. Subsequently a signal of the phosphorus standard and of the second peptide is detected in LC-ICP-MS. The concentration of the second peptide is determined by comparison of the peak areas of the corresponding phosphorus signals within the LC-ICP-MS run. According to an embodiment of the invention a previously determined correction factor is used to account for signal intensity changes due to gradient elution (see Wind M. et al. Anal. Chem. 2001, 73, 29-35).

The phosphorus standard having known phosphorus concentration can be selected from various commercially available standards. According to an embodiment of the invention, a bis-4-nitrophenylphosphate (BNPP) standard of known concentration is used in the method of the invention. According to a further aspect of the invention aromatic mono- and diesters of phosphoric acid having a known concentration can be used as phosphorus standard.

After absolute quantification of the second peptide, an aliquot comprising the second peptide in known concentration is completely dephosphorylated to obtain a third peptide with known concentration. According to the invention, the third peptide is a completely dephosphorylated and isotope labeled form of the first peptide. For the purpose of dephosphorylation the second peptide is treated with a phosphatase e.g. selected from alkaline phosphatase and antarctic phosphatase to hydrolyse the phosphate ester bond(s) within the phosphopeptide. According to a further aspect of the invention, the second peptide can be dephosphorylated by the use of a reagent that contains at least one selected from the group consisting of hydrogen fluoride, hydrofluoric acid, and a hydrogen fluoride-containing compound. The use of the above mentioned reagents leaves the second peptide intact and is applicable to any kind of the type. This method is disclosed in the European patent application EP 1 595 889 A1. According to the embodiment of the invention if the "first peptide" does not contain a S, T or Y residue an enzyme cleavable extension can be introduced carrying a S, T or Y residue to obtain the second peptide which is quantified by LC-ICP-MS. The third peptide is then derived by treatment of the second peptide with a protease.

When phosphatase was used for dephosphorylation of the second peptide, usually a small amount of phosphatase remains in the sample containing the third peptide. In this case, prior combining a known amount of the second peptide and a known amount of the third peptide to obtain the reference sample, the phosphatase used for dephosphorylation has to be inactivated, to retain the accuracy of the quantification. Usually the used phosphatase was inactivated by acidification to pH ≤ 3. For the preparation of acid-labile peptides, a different phosphatase, such as antarctic phosphatase, can be used. This phosphatase can be inactivated irreversibly by moderate heating (e.g. 5 min at 65°C or extended incubation times at 55°C). In this way, acidification of the solution and possible hydrolysis of acid-labile peptides is avoided.

According to the invention, the reference sample containing a known amount of the second peptide (isotope labeled phosphopeptide) and a known amount of the third peptide (corresponding isotope labeled unphosphorylated peptide) is combined with the sample containing the first peptide, and molecular ion and/or fragment ion signals of each of the peptides are detected in an analyzer that is an ESI, LC-ESI-MS or MALDI analyzer. Since the isotope label of the second peptide leads to a mass shift, the signals of the three peptides can be identified by use of an MS analyzer. Since the amount of phosphorylated and unphosphorylated second and third peptide is known, the degree of phosphorylation of the first peptide can be determined by comparing the respective molecular or fragment ion signals. According to a further aspect of the invention, the absolute concentration of the first peptide is determined by comparison of respective molecular or fragment ion signals.

The invention further refers to a reference sample containing a second peptide which contains at least one phosphate group and a known degree of phosphorylation and which is an isotope labelled form of a first peptide, and a third peptide, that is a completely dephosphorylated and isotope labelled form of the first peptide derived from the second peptide in a known concentration prepared obtained by a method comprising:
(b) selecting a first peptide
(ba) providing a phosphorus standard having a known phosphorus concentration,
(bb) providing the second peptide and combining the second peptide and the phosphorus standard,
(bc) detecting a signal of the second peptide and of the phosphorus standard in an LC-ICP-MS,
(bd) determining the concentration of the second peptide, by comparing the respective signals,
(be) providing an aliquot of the second peptide of known concentration obtained in step (bd) and hydrolysing the phosphoric acid ester bond(s) to obtain the third peptide with known concentration, and
(bf) combining a known amount of the second peptide quantified in step (bd) and a known amount of the corresponding completely dephosphorylated third peptide obtained in step (be) to obtain the reference sample,
   and the use of the reference sample for determining the degree of phosphorylation and/or absolute amount of a phosphopeptide.

The method of the invention can be used in a variety of different applications. For example, the method of the invention can be used for profiling blood serum. The possibility to analyze readily accessible specimens such as blood serum is particularly useful in clinical applications. Hence, the method is also applicable to basic biology and clinical analysis.

The accuracy of the method of the invention is shown in table 1. Table 1 shows the recoveries of experiments, where three calibrated solutions of sulfur-containing phosphopeptides were prepared via the method described above. In all three independent experiments phosphorus and sulfur were monitored by LC-ICP-MS. The concentrations of the phosphopeptides (also determined by S detection) and of the dephosphorylated peptides (determined by S detection) were found to be identical within the experimental error. These data support the practicability of the concept introduced, namely the preparation of calibrated peptide solutions via dephosphorylation of phosphopeptides.

**Table 1. Recovery of different peptides after treatment of phosphopeptides with alkaline phosphatase.**

| **Sequence** | **Recovery after dephosphorylation** |
|---|---|
| HTDDEMpTGpYVATR | 103 % ± 3% |
| YRSVpTPCDM | 93% ± 6% |
| YRpSVTPCDM | 100 % ± 3% |

The method of the invention is advantageous because it incorporates significantly less steps and thus contains less sources of error. A single analysis requires 0.01 to 0.1 nmol of the first peptide and at least three replicates are recommended. A detection limit of the preferably used LC-ICP-MS analyzer is below 0.1 pmol, a sample amount ≥ 10 pmol is recommended to obtain a reproducible and accurate quantification in a single analysis. Further, the quantification by LC-ICP-MS with subsequent phosphatase treatment is less time consuming than the classical aforementioned amino acid analysis connected with the AQUA quantification method and less sensitive to common buffers and detergents used during peptide synthesis. Additionally, the quantification is carried out by a peptide-specific signal, which ensures that only the synthetic peptide is quantified while any impurities resolvable by LC are not measured. In contrast, amino acid analysis quantifies all amino acids and peptides present in the sample besides the selected synthetic peptide.

The following Figures and examples are included to further illustrate various aspects of the present invention. It should be appreciated by those of skilled in the art that the techniques disclosed in the examples which follow the presented techniques and/or compositions discovered by the inventor to function well in the practise of the invention, and thus can be considered to constitute preferred modes for its practice. However, those of skilled in the art should, in light of a present disclosure, appreciate that many changes can be made in the specific embodiments which are disclosed and still obtain alike or a similar result without departing from the spirit and the scope of the invention.

### Figures:

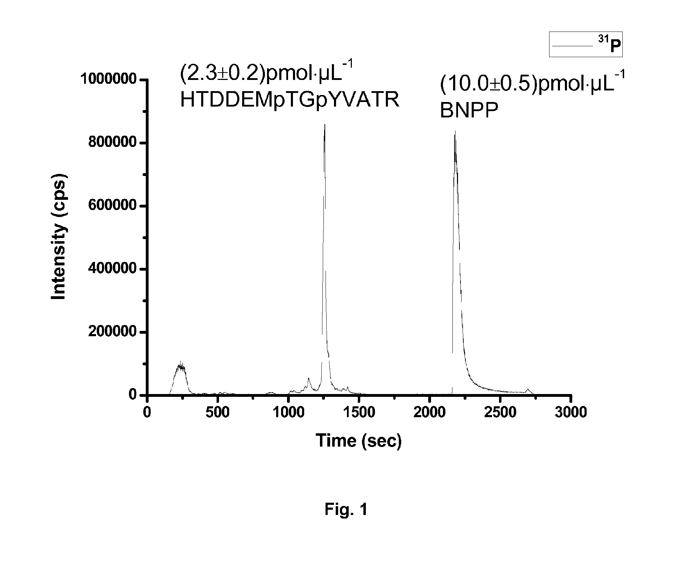

Fig: 1: Figure 1 shows the principle of absolute quantification. The signal areas of the second peptide (HTDDEMpTGpYVATR) and of the internal standard (BNPP) are compared. Based on the known amount of the internal standard, the absolute concentration of the phosphopeptide is calculated.
Fig. 2: Figure 2 shows the results of an LC-ICP-MS analysis (detection of P and S) of the second peptide (HTDDEMpTGpYVATR) before phosphatase treatment. The first major phosphorus peak corresponds to the peptide, the second major peak to the internal standard BNPP.
Fig. 3: Figure 3 shows the results of an LC-ICP-MS analysis (detection of P and S) of the second peptide (HTDDEMpTGpYVATR) after phosphatase treatment. It is shown that phosphate is completely removed from the second peptide. Removal of the two phosphate residues results in a shift to a longer retention time. The sulphur is solely used to prove the quantitative nature of the phosphatase reaction and to demonstrate the resulting preservation of the sample concentration by LC-ICP-MS. The presence of sulphur is not essential for the described procedure.
Fig. 4: Figure 4 shows the elution of the phosphopeptide HTDDEMpTGpYVATR analyzed by LC-ICP-MS and ³²S detection.
Fig. 5: Figure 5 shows the analysis of the same sample as in Figure 4, but following treatment with phosphatase.
Fig.6: Figure 6 shows the chromatogram of an equimolar mixture of phosphatase treated and untreated phosphopeptide as shown in Fig. 4 and Fig. 5 after 20 h incubation in an acidic solution. The signal of the phosphopeptide is not altered during the 20 h incubation time, as can be concluded from the identical signal intensity compared to the unphosphorylated analog. This shows that the phosphatase inactivation is complete and additionally that the procedure of phosphatase treatment preserves the previously determined absolute concentration of the peptide including all essential dilution steps.

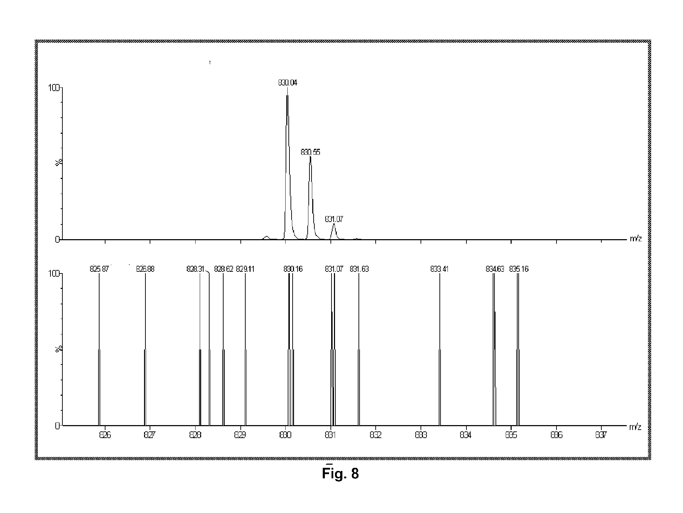

Fig. 7: Figure 7 shows nanoESI mass spectra of the phosphopeptide HTDDEMpTGpYV-[¹⁵N, ¹³C₃]A-TR; A) before, and B) after treatment with antarctic phosphatase. The complete removal of both phosphate groups from the peptide is shown in the marked area (see Fig. 8).
Fig. 8: Figure 8 shows an expanded area of the spectrum given in Fig. 7 as indicated. The signal of the doubly phosphorylated peptide is not detectable following dephosphorylation.
Fig. 9: Figure 9 shows the probability for the occurrence of at least one serine, threonine or tyrosine residue as a function of the peptide length, thus showing the almost general applicability of the method.

In Figure 1, the principle of absolute quantification is shown. For this purpose, the solution of the phosphopeptide HTDDEMpTGpYVATR is subjected to absolute quantification by liquid chromatography elemental mass spectrometry (LC-ICP-MS) and measurement of phosphorus. For this purpose a known amount of the internal standard bis-4-nitrophenylphosphate (BNPP) is added in a concentration of 10.5 ± 0.5 pmol/µl The quantification is carried out by comparison of the peak areas of the corresponding phosphorus signals within the LC-ICP-MS run. A previously determined correction factor is used to account for signal intensity changes due to gradient elution (see Wind M, et al., Anal. Chem. 2001, 73, 29-35).

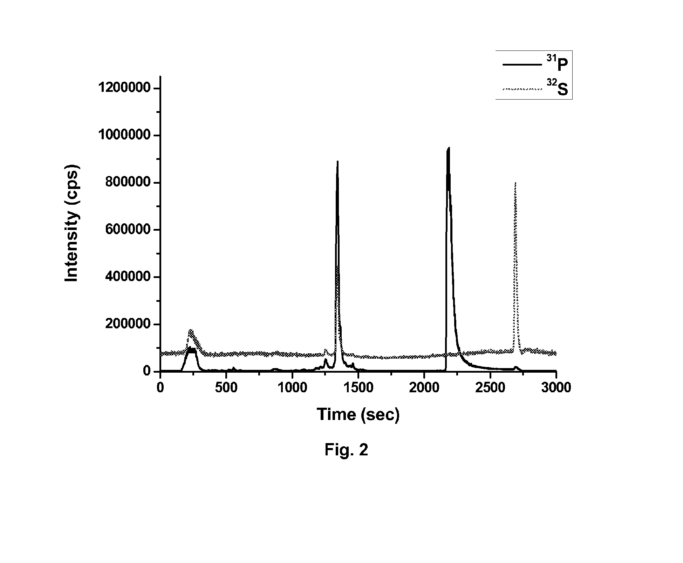

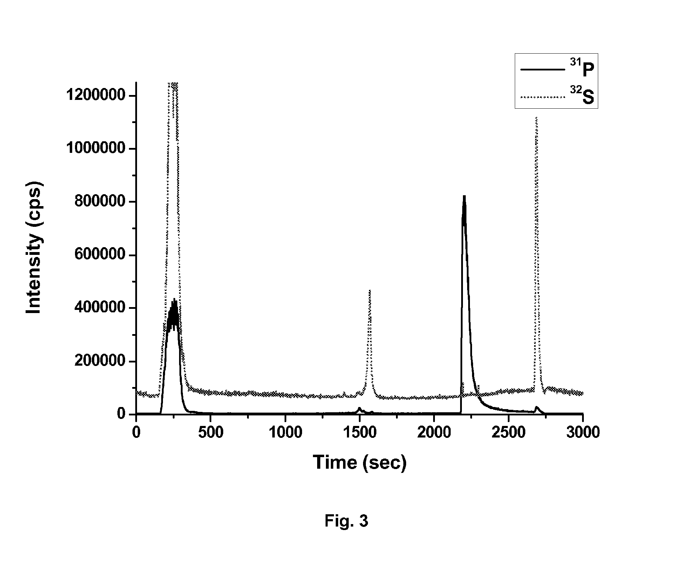

Figures 2 and 3 show an LC-ICP-MS analysis (detection of P and S) of the phosphopeptide (HTDDEMpTGpYVATR) before (Fig. 2) and after (Fig. 3) phosphatase treatment. To ensure the quantitive enzymatic dephosphorylation, sulphur and phosphorus are monitored simultaneously by LC-ICP-MS within the peptide before and after phosphatase treatment. As shown in Figure 3, phosphate is completely removed from the phosphopeptide, while the intensity of the sulphur signal is left unchanged proving that phosphatase treatment prevents the absolute amount of peptide. The retention time is increased by dephosphorylation, since the dephosphorylated peptide exhibits a different chromatographic behaviour.

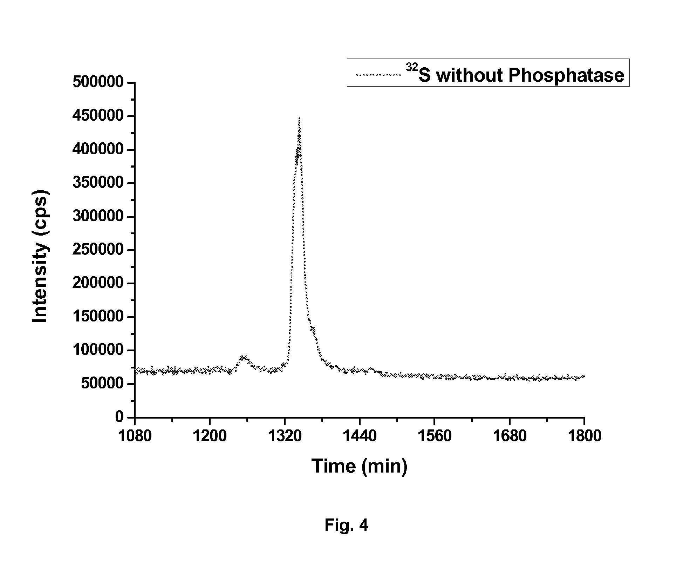

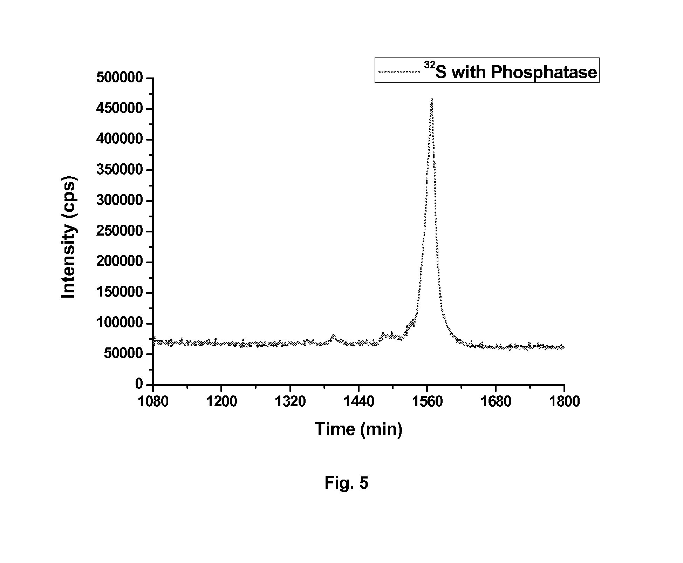

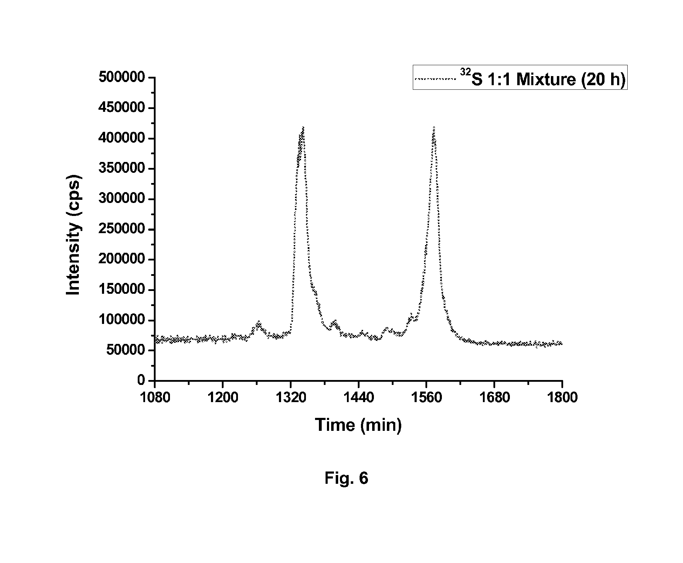

After enzymatic dephosphorylation, a small amount of phosphatase used for dephosphorylation remains in the calibrated solution. To ensure a general applicability of this solution as internal standard, the phosphatase has to be inactivated. Alkaline phosphatase was inactivated by acidification to pH ≤ 3. To confirm the inactivation of the alkaline phosphatase an equimolar solution of phosphatase-treated and untreated phosphopeptide was prepared and analysed after an incubation time of about 20 hours (see Figure 6). The sulphur signals corresponding to the phosphorylated (Fig. 4) and unphosphorylated peptide (Fig. 5) are not altered during the incubation time proving complete inactivation of the alkaline phosphatase.

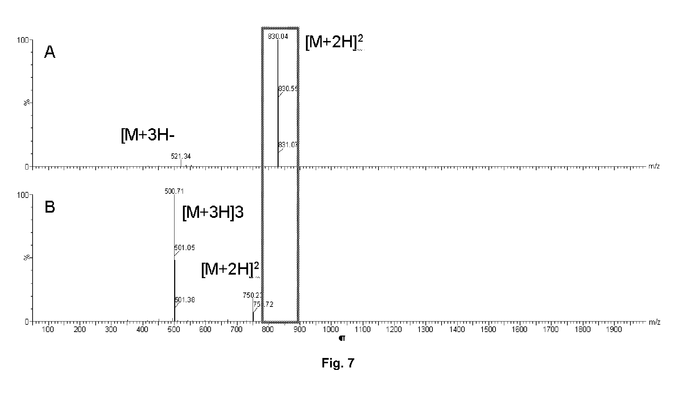

For the preparation of absolutely quantified acid-labile peptides, a different phosphatase, such as antarctic phosphatase can be used. This phosphatase can be inactivated irreversibly by moderate heating (e.g. 5 min at 65°C or extended incubation times at 55°C). In this way, acidification of the solution and possible hydrolysis of acid-labile peptides is avoided. nanoESI mass spectra of a phosphopeptide solution before and after antarctic phosphatase treatment are shown in Figure 7. In this case, a stable isotope labelled phosphopeptide (second peptide) was treated with antarctic phosphatase to a stable isotope labelled peptide (third peptide) further proving the quantitative reaction.

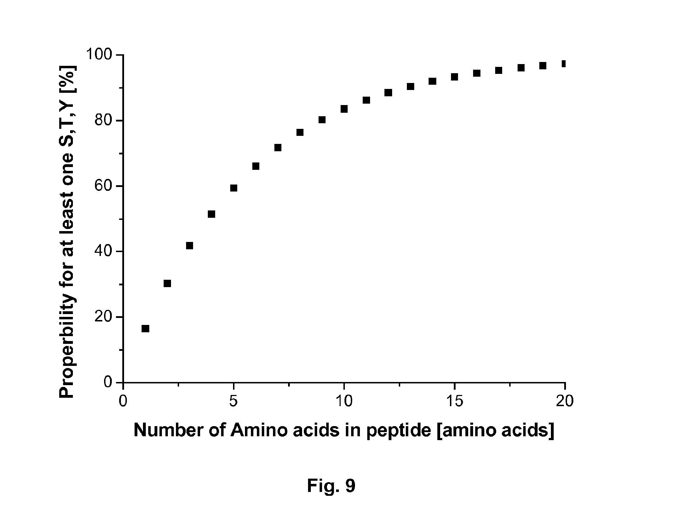

The method of the invention is based on the presence of serine, threonine and tyrosine residues within the selected peptide (first peptide). The probability for the presence of at least one of these residues in an average peptide sequence is shown in Figure 9. The calculation is based on the average abundance of serine (7%), threonine (6.1%) and tyrosine (3.4%) in mammalian proteins. The plot in Figure 9 shows that already for peptides with a length of 12 residues, about 90% of the sequences statistically contain one of the residues serine, theronine or tyrosine, which can be introduced into synthetic peptides in phosphorylated form.

## Claims

1. A method for determining the degree of phosphorylation of a first peptide, which is present in a sample in phosphorylated and/or unphosphorylated form or any mixtures thereof comprising:
(a) obtaining a sample containing said first peptide,
(b) preparing a reference sample containing a second peptide which contains at least one phosphate group and a known degree of phosphorylation and which is an and isotope labelled form of the first peptide, and a third peptide, that is a completely dephosphorylated and isotope labelled form of the first peptide in a known concentration comprising:
(ba) providing a phosphorus standard having a known phosphorus concentration,
(bb) providing the second peptide and combining the second peptide and the phosphorus standard,
(bc) detecting a signal of the second peptide and of the phosphorus standard in an LC-ICP-MS system,
(bd) determining the concentration of the second peptide, by comparing the respective phosphorus signals,
(be) providing an aliquot of the second peptide of known concentration obtained in step (bd) and hydrolysing the phosphoric acid ester bond(s) to obtain the third peptide with known concentration, and
(bf) combining a known amount of the second peptide quantified in step (bd) and a known amount of the corresponding completely dephosphorylated third peptide obtained in step (be) to obtain the reference sample,
(c) combining the reference sample and the sample containing the first peptide,
(d) detecting the molecular ion and/or fragment ion signals of each of the peptides and differentiating the signals with an mass analyzer, and
(e) determining the degree of phosphorylation of the first peptide by comparison of the respective mass spectrometric signals.

2. The method according to claim 1, wherein in a further step (f) the absolute amount of the first peptide is determined by comparison of the respective mass spectrometric signals.

3. The method according to claim 1 or 2, wherein the mass analyzer used in step (d) is selected from an ESI, LC-ESI and MALDI mass spectrometer.

4. The method according to any of claims 1 to 3, wherein the first peptide is a pharmaceutically active phosphopeptide.

5. The method according to any of claims 1 to 4, wherein the first peptide is obtained from a cell, a prokaryotic cell, an eukaryotic cell, a mammalian cell, a human cell, from an organ or a human organ, from plasma or from serum.

6. The method according to any of claims 1 to 5, wherein in step (bf) a phosphatase is used to hydrolyze the phosphoester bonds and wherein the phosphatase is inactivated after the hydrolysis.

7. The method according to any of claims 1 to 5, wherein in step (bf) one selected from the group consisting of hydrogen fluoride, hydrofluoric acid and a hydrogen fluoride-containing compound is used in to hydrolyze the phosphoester bonds.

8. The method according to any of claims 1 to 7, wherein the isotope labelled form of the first peptide comprises one or more isotopes selected from the group consisting of ¹³C, ¹⁵N, ²H, ¹⁸O and ³⁴S_{.}

9. The method according to any of claims 1 to 8, wherein bis-4-nitrophenylphosphate (BNPP) or any other phosphate standard of known concentration is used as phosphorus standard in step (bb).

10. A reference sample containing a second peptide which contains at least one phosphate group and a known degree of phosphorylation and which is an isotope labelled form of a first peptide; and a third peptide, that is a completely dephosphorylated and isotope labelled form of the first peptide derived from the second peptide in a known concentration prepared obtained by a method comprising:
(b) selecting a first peptide
(ba) providing a phosphorus standard having a known phosphorus concentration,
(bb) providing the second peptide and combining the second peptide and the phosphorus standard,
(bc) detecting a signal of the second peptide and of the phosphorus standard in an LC-ICP-MS,
(bd) determining the concentration of the second peptide, by comparing the respective signals,
(be) providing an aliquot of the second peptide of known concentration obtained in step (bd) and hydrolysing the phosphoester bonds to obtain the third peptide with known concentration, and
(bf) combining a known amount of the second peptide quantified in step (bd) and a known amount of the corresponding completely dephosphorylated third peptide obtained in step (be) to obtain the reference sample,

11. Use of the reference sample according to claim 10 for determining the degree of phosphorylation of a phosphopeptide.

12. Use of the reference standard according to claim 11 for determining the absolute amount of a phosphopeptide.
